# EUROPÄISCHE PATENTSCHRIFT

(11) **EP 3 192 507 B1**
(45) Veröffentlichungstag und Bekanntmachung des Hinweises auf die Patenterteilung: **08.07.2020**
(21) Anmeldenummer: 17158393.3
(22) Anmeldetag: 10.05.2007
(51) Int. Cl.: A61K 31/4174, A61K 45/06, A61P 31/10, A61P 29/00

(54) **VERWENDUNG VON KOMBINATIONSPRÄPARATEN, UMFASSEND ANTIMYKOTIKA**
USE OF COMBINATION PREPARATIONS COMPRISING ANTIMYCOTIC AGENTS
UTILISATION DE PRÉPARATIONS COMBINÉES COMPRENANT DES FONGICIDES

(30) Priorität: 12.05.2006 AT 8262006
(43) Veröffentlichungstag der Anmeldung: 19.07.2017
(62) Teilanmeldung aus: 07718439.8
(73) Patentinhaber: ProFem GmbH, 1180 Wien (AT)
(72) Erfinder: NOE, Christian, 1180 Wien (AT); NOE-LESCHNIG, Marion, 1180 Wien (AT)
(74) Vertreter: Sonn & Partner Patentanwälte

(56) Entgegenhaltungen:
- EP-A- 1 637 132
- EP-A- 1 652 535
- EP-A1- 0 592 348
- WO-A-00/56353
- WO-A-95/17165
- WO-A-2005/117831
- WO-A1-00/48633
- US-A1- 2003 181 384
- US-A1- 2004 142 910
- US-A1- 2005 014 729
- YUCESOY M ET AL: "In-vitro synergistic effect of fluconazole with nonsteroidal anti-inflammatory agents against Candida albicans strains", JOURNAL OF CHEMOTHERAPY, Bd. 12, Nr. 5, Oktober 2000 (2000-10), Seiten 385-389, XP008077767, ISSN: 1120-009X
- SCOTT E M ET AL: "Demonstration of synergy with fluconazole and either ibuprofen, sodium salicylate, or propylparaben against Candida albicans in vitro", ANTIMICROBIAL AGENTS AND CHEMOTHERAPY 1995 UNITED STATES, Bd. 39, Nr. 12, 1995, Seiten 2610-2614, XP002196358, ISSN: 0066-4804
- TARIQ V N ET AL: "Use of decimal assay for additivity to demonstrate synergy in pair combinations of econazole, nikkomycin Z, and ibuprofen against Candida albicans in vitro", ANTIMICROBIAL AGENTS AND CHEMOTHERAPY, AMERICAN SOCIETY FOR MICROBIOLOGY, WASHINGTON, DC, US, Bd. 39, Nr. 12, Dezember 1995 (1995-12), Seiten 2615-2619, XP002196359, ISSN: 0066-4804
- LÉVY G: "[Value of benzydamine, the first anti-inflammatory vaginal solution]", REVUE FRANÇAISE DE GYNÉCOLOGIE ET D'OBSTÉTRIQUE NOV 1989, Bd. 84, Nr. 11, November 1989 (1989-11), Seiten 779-781, XP000676492, ISSN: 0035-290X
- FILLER S G ET AL: "Candida albicans stimulates cytokine production and leukocyte adhesion molecule expression by endothelial cells", INFECTION AND IMMUNITY 1996 UNITED STATES, Bd. 64, Nr. 7, 1996, Seiten 2609-2617, XP008077756, ISSN: 0019-9567
- CANNOM R R M ET AL: "Candida albicans stimulates local expression of leukocyte adhesion molecules and cytokines in vivo", JOURNAL OF INFECTIOUS DISEASES 01 AUG 2002 UNITED STATES, Bd. 186, Nr. 3, 1. August 2002 (2002-08-01), Seiten 389-396, XP008077808, ISSN: 0022-1899
- KLOTZ S A: "Adherence of Candida albicans to endothelial cells is inhibited by prostaglandin I2", INFECTION AND IMMUNITY 1994 US, Bd. 62, Nr. 4, 1994, Seiten 1497-1500, XP008096038, ISSN: 0019-9567
- HOFFMAN H L ET AL: "Review of the safety and efficacy of voriconazole", EXPERT OPINION ON INVESTIGATIONAL DRUGS 2002 GB, Bd. 11, Nr. 3, 2002, Seiten 409-429, XP008096052, ISSN: 1354-3784
- KRUSZEWSKA H ET AL: "SEARCH OF ANTIMICROBIAL ACITIVITY OF SELECTED NON-ANTIBIOTIC DRUGS", ACTA POLONIAE PHARMACEUTICA, POLISH PHARMACEUTICAL SOCIETY, WARZSAW, PL, Bd. 59, 1. Januar 2002 (2002-01-01), Seiten 436-439, XP008065730, ISSN: 0001-6837

## Beschreibung

Die Erfindung betrifft die Verwendung von Kombinationspräparaten, umfassend Antimykotika, zur Behandlung von Candidamykosen. Hefen, v.a. Candida spp. (Candida albicans) sind für gewöhnlich opportunistische Krankheitserreger, welche erst bei einer Schwächung der systemischen bzw. lokalen Immunabwehr pathogen werden (z.B. Mundsoor bei HIV-Erkrankten, Leukämiepatienten, unter Chemotherapie, bei Säuglingen; intertriginöses Ekzem bei Diabetes mellitus). Eine Ausnahme hiervon bildet die vulvovaginale Candidamykose der ansonsten gesunden Frau. Allerdings gibt es auch bei der vaginalen Candidose eine Reihe von Faktoren, die eine Erhöhung der Virulenz des Erregers bewirken, und bei entsprechender persönlicher Disposition von einer asymptomatischen Besiedelung zu einer symptomatischen Erkrankung führen können.

Bei der Vulvovaginalmykose handelt es sich um eine sehr häufige Erkrankung, die in den letzten Jahren auch stetig zugenommen hat. Nach neueren Schätzungen erkranken drei von vier Frauen wenigstens einmal in ihrem Leben an einer Vaginalmykose. In 5% der Fälle kommt es zu einem chronisch-rezidivierenden Verlauf, der für die betroffene Patientin oft eine schwere subjektive Beeinträchtigung darstellt. Besondere Schwierigkeiten bietet die Behandlung der vaginalen Mykose in der Schwangerschaft, da in diesem Fall offenbar besonders begünstigende Faktoren von Seiten der Patientin vorliegen, andererseits die Therapiemöglichkeiten wesentlich eingeschränkt sind und drittens die absolute Therapienotwendigkeit besteht, da es ansonsten während der Geburt zu einer Infektion des Neugeborenen kommt.

Oro-pharyngeale Soor-Erkrankungen wiederum sind nicht nur bei Neugeborenen mit ihrem unreifen Immunsystem ein Problem, sondern auch bei Patienten mit geschwächter Immunabwehr, wie HIV- Patienten, Patienten unter Chemotherapie und bei Patienten mit hämatologischen Malignomen. Es ist - wie erwähnt - bekannt, dass Infektionskrankheiten geschwächte und immunsupprimierte Menschen besonders leicht befallen, wobei - wie im Fall vieler Erkrankungen durch Candida spp. - aus harmlosen Symbionten Krankheitserreger werden.

Dennoch wurde diese Tatsache im therapeutischen Konzept bisher nicht ausreichend mechanistisch berücksichtigt. Bisher ist kein Arzneimittel, bzw. keine Kombination von Arzneistoffen in einem Arzneimittel, zugelassen worden, bei welchem erklärter Maßen zugleich mit dem unmittelbaren "Angriff" auf den Pilz auch durch Beeinflussung physiologischer Reaktionen des Patienten die Infektiosität des Erregers auf zellulärer Ebene reduziert bzw. unterbunden wird.

Die Therapie der cutanen und mukocutanen Candidiasis erfolgt derzeit ausschließlich durch Antimykotika, welche, abhängig von der Dosierung, fungistatisch oder fungicid wirken und, je nach Wirkstoff, systemisch oder lokal aufgebracht werden.

Zur Therapie von Candidamykosen, wie des Mundhöhlensoors oder der Vaginalmykose, ist eine Vielzahl von Arzneimitteln mit antimykotischer Wirkung zugelassen, meist für die topische, aber auch für die systemische Therapie. Der Mechanismus der pharmakologischen Wirkung fast aller verwendeten Arzneistoffe ist wohl bekannt. Naheliegender Weise orientieren sich die meisten bekannten Mechanismen am infizierenden Organismus (Bakterium oder Pilz) selbst. So greift eine Reihe dieser Arzneistoffe hemmend in die Ergosterol-Biosynthese (Isopren-Biosyntheseweg) ein. Der Arzneistoff versucht, den Pilz über physiologische Vorgänge, welche sich möglichst vom menschlichen Stoffwechsel unterscheiden, zu hemmen oder abzutöten.

In der WO 00/56353 A2 werden von alpha-Metanocyten-stimulierendem Hormon abgeleitete Peptide zur Behandlung von Candidavaginitis vorgeschlagen.

Die EP 1 637 132 A1 betrifft Zusammensetzungen zur Behandlung von Fußpilz bei Sportlern.

Die EP 0 592 348 A1 offenbart Zusammensetzungen zur Behandlung opthalmischer Erkrankungen.

In der WO 2005/117831 A1 werden Posaconazol-enthaltende Präparate zur Injektion bei Pilzbehandlungen beschrieben.

Gemäß der WO 00/48633 A1 werden Antikörper gegen hydrophobe Proteine zur Bekämpfung von Candidamykosen vorgeschlagen.

Die US 2003/181384 A1 betrifft die Verabreichung von Trefoil-Peptiden zur Verhinderung von Epithelschäden, die durch Infektionen verursacht sein können. Lokale anti-inflammatorische Vaginallösungen enthaltend Benzydamin zur mechanischen Entfernung von Pilzmassen werden von Levy (Rev.fr.Gynecol.Obstet. 84 (1989), 779-781) vorgeschlagen.

Die WO 95/17165 A2 offenbart ein Gel dass Clotrimazol und Ibuprofen enthält (Beispiel III).

Für die Candidavaginitis, v.a. bei chronischem Verlauf, ergibt sich aus dem vorhandenen Wirkstoffspektrum derzeit keine befriedigende Therapiemöglichkeit. Dies gilt in besonderem Maß, falls die Erkrankung während der Schwangerschaft auftritt.

Die molekularen Faktoren, die die persönliche Disposition begründen, sind im Detail unbekannt, dementsprechend existiert bisher kein Arzneimittel, bzw. keine Kombination von Arzneistoffen in einem Arzneimittel, bei welchem zugleich mit dem unmittelbaren "Angriff" auf den Pilz auch durch Beeinflussung physiologischer Reaktionen des Patienten die Infektiosität des Erregers auf zellulärer Ebene reduziert bzw. unterbunden wird. Es besteht daher ein dringendes Bedürfnis nach Therapien für Candidamykosen, die bislang durch konventionelle Antimykotika-Verabreichung nicht erfolgreich behandelt werden können. Insbesondere sollen dabei Candidamykosen behandelt werden, die einerseits Schleimhautinfektionen betreffen, vor allem vulvovaginale Candidiasis, oropharyngeale Candidiasis (Mundsoor), andererseits Candidamykosen auf vorgeschädigter (mazerierter) Haut, wie Windeldermatitis (Windelsoor) und intertriginöses Ekzem. Dabei sollte eine derartige Therapie auch die Behandlung von Vulvovaginalmykosen bei Schwangeren ermöglichen, welche einen besonders behutsamen Einsatz von Arzneimitteln verlangt.

Demgemäß betrifft die vorliegende Erfindung Verwendung von
- einem antimykotischen Wirkstoff und
- einem Epithelzellen- oder Endothelzellen-Adhäsionsinhibitor zur Herstellung eines Kombinationsarzneimittels zur topischen Behandlung von Candidamykosen ausgewählt aus vulvovaginaler Candidiasis, oropharyngealer Candidiasis (Mundsoor), Windeldermatitis (Windelsoor) und intertriginösem Ekzem, wobei der Epithelzellen- oder Endothelzellen-Adhäsionsinhibitor ausgewählt ist aus einem nicht-steroidalen Antiphlogistikum (NSAID) mit einer ausreichenden COX1-Hemmung, definiert durch den Ausschluss von Verbindungen mit einem COX1/COX-2-Verhältnis (IC50, microM) von > 20, wobei das Kombinationsarzneimittel eine Salbe, eine Creme, ein Vaginal-Suppositorium, eine Vaginal-, Buccal- oder Sublingual-Tablette, ein Puder, oder ein Aerosol ist.

Den erfindungsgemäß bevorzugt zu behandelnden Pilzerkrankungen ist gemeinsam, dass nicht nur von Erregerseite, sondern auch von Seiten des betroffenen Organismus (des Patienten) wesentliche Beiträge zur Krankheitsentstehung und Aufrechterhaltung der Infektion geleistet werden. Abgesehen von der grundsätzlich erhöhten Neigung zur Erkrankung bei einem geschwächten Immunsystem des Patienten, sind es die Mechanismen der Infektiosität, welche in diesem Zusammenhang von besonderer Bedeutung sind. Erst in den letzten Jahren werden derartige Konzepte vermehrt in der Antiinfektiva-Forschung untersucht.

Der erste, die Infektiosität bestimmende Schritt beim Befall einer Zelle durch einen Erreger besteht stets im Andocken des Erregers an die Plasmamembran einer Zelle. Generell ist für das Andocken im Sinne einer Zell-Zell-Interaktion eine Vielzahl verschiedener Systeme von Adhäsionsmolekülen bekannt geworden. Die generelle Fähigkeit von Krankheitserregern zum Anhaften an Epithelien und Endothelien verläuft ebenso über die Interaktion mit Adhäsionsmolekülen. Für bestimmte Erreger, für bestimmte Organe und bei bestimmten Krankheitsbildern ist ein spezieller Satz der involvierten Adhäsionsmoleküle zu erwarten. Für Infektionen im Allgemeinen und Pilzinfektionen im Besonderen sind jedoch jeweils nur bruchstückhaft beteiligte Adhäsionsfaktoren publiziert, ohne jedoch zu einem umfassenden Konzept zusammengefasst worden zu sein oder Eingang in ein therapeutisches Konzept gefunden zu haben. Der vorliegenden Erfindung liegt der Befund zugrunde, dass die gleichen Adhäsionsmoleküle, welche im Zuge der Blutgerinnung bei der Anhaftung der Thrombozyten eine entscheidende Rolle spielen und deren Expression durch Abkömmlinge des Arachidonsäurestoffwechsels induziert wird (unter anderem von Willebrand-Faktor, Vitronektin, Fibronektin, Integrine), im Rahmen der Transformation zur Pathogenität von Begleitfaktoren oder dem Erreger selbst induziert werden und nachfolgend von den primär opportunistischen Erregern für die Anhaftung ausgenützt werden.

Es ist seit längerem bekannt, dass Candidapilze unter dem Einfluß von Prostaglandinen eine Transformation ihrer Gestalt von der Sproßform zur Hyphenform erfahren, welche mit vermehrtem Wachstum einhergeht. Prostaglandine sind Abkömmlinge der Arachidonsäure. Das Wirkprinzip der Arzneistoffklasse der NSAID, welche vor allem zur Behandlung entzündlicher und rheumatischer Erkrankungen eingesetzt werden, besteht primär in der Hemmung der Prostaglandinbiosynthese. Der Einfluß von NSAID auf das Wachstum von Pilzen wurde in in-vitro-Experimenten (Scott et al., Antimicrob. Ag. Chemother. 39 (12) (1995), 2610-2614; Tariq et al., Antimicrob. Ag. Chemother. 39 (12) (1995), 2615-2619; Yucesoy et al., J.Chemother. 12 (2000), 385-389) aufgezeigt. Bei dieser Kombination mit Antimykotika wurde gelegentlich eine Verstärkung des antimykotischen Effektes beobachtet, jedoch hat sich dafür ebenso explizit die Bezeichnung "paradoxer Effekt" etabliert, weil sich bei manchen Konzentrationen und bei manchen Kombinationen in vitro statt einer Hemmung ein verstärktes Wachstum ergibt (Arai et al, Mycoses 48 (2005), 38-41). So zeigten die Effekte von Ibuprofen in Kombination mit verschiedenen Antimykotika starke Unterschiede (Tariq et al., 1995). Fluconazol-sensible Stämme von C. albicans zeigten im in-vitro-System kein verbessertes Ansprechen auf eine Kombination von Ibuprofen mit Fluconazol (Arai et al., 2005). Die Ergebnisse der in-vitro-Versuche veranschaulichen insgesamt, dass bezogen auf das Pilzwachstum die Effekte vom jeweiligen verwendeten Pilzstamm abhängen, jedoch daraus keine Rückschlüsse auf die medizinische Wirksamkeit am Ort der Pilzinfektion ableitbar sind und zwar weder in die eine (positive) noch in die andere (negative) Richtung.

Da bei üblichen in-vitro- Experimenten über antimykotische Wirkungen bestimmter Stoffe oder Stoffkombinationen die physiologische Antwort des Wirtes außer Betracht bleibt, kann in keiner dieser "in-vitro"-Arbeiten auf die Rolle der Zell-Zell-Adhäsion bei der Infektion im Allgemeinen oder auf Veränderungen des Epithels bzw. Endothels des Wirtes auch nur im geringsten eingegangen werden.

Darüber hinaus wurde bisher in der einschlägigen Literatur die Therapieresistenz bei der Behandlung der chronisch-rezidivierenden Candidavulvovaginitis unter anderem auf das gehäufte Auftreten bestimmter Candidasubspecies zurückgeführt (ohne jedoch ein schlüssiges Gesamtkonzept zu bieten). Gerade für diese Subspecies liegen keinerlei in-vitro-Ergebnisse vor.

Voraussetzung für das schichtförmige Überwuchern ist z.B. im Fall der Candida tatsächlich die Transformation der Wuchsform von der Sproß- auf die Hyphenform. Dadurch verstärkt sich die Pathogenität der Erreger deutlich. Die Bildung der Hyphen wird ihrerseits durch Prostaglandin E2 gefördert, welches im Rahmen des Entzündungsgeschehens auch von der Endothelzelle/Epithelzelle des Patienten gebildet wird. Auch die beim entzündlichen Geschehen vermehrte Mucusbildung von Schleimhäuten spielt bei diesem Krankheitsgeschehen eine Rolle.

Unter bestimmten begünstigenden Bedingungen im Mikroenvironment, wie z.B. bei geschwächter Immunlage, aber auch bei geeigneten hormonellen Bedingungen oder genetischer Disposition eines Patienten oder andererseits aufgrund von "quorum sensing", können manche Krankheitserreger, wie im Speziellen auch Candidaarten, den betroffenen Haut-, Schleimhaut oder Endothelbezirk unter Bildung einer geschlossenen Schicht überwuchern. Dies führt nun nicht nur zur Aggravierung des Krankheitsgeschehens. Durch die Transformation der Wuchsform ändert sich auch die Angreifbarkeit der Erreger durch lokale Behandlungsmethoden. Die Erreger sind in dieser Wuchsform durch die üblichen lokal applizierten Antimykotika praktisch nicht mehr angreifbar. Es kommt zur Chronifizierung und es wird notwendig, von einer Lokaltherapie zu einer systemischen Therapie überzugehen. Eine systemische antimykotische Therapie ist aber wegen des Nebenwirkungsspektrums vieler Antimykotika häufig sehr belastend, im Fall von Schwangeren wegen teratogener Wirkungen gänzlich ausgeschlossen. Doch auch unter systemischer Therapie kommt es bei der durch Candida spp. (mit Candida albicans und Candida glabrata als häufigsten Erregern) verursachten chronisch-rezidivierende Vulvovaginitis und Kolpitis häufig dennoch nicht zur Heilung und bei vielen Patientinnen zu mehrfachen Rezidiven im Jahr.

Der vorliegenden Erfindung liegt der bisher unbekannte, aber für die Infektiosität zentrale Befund zugrunde, dass sich Hefepilze beim Andocken an Epithelzellen (bzw. Endothelzellen) der Arachidonsäure-basierten körpereigenen Mechanismen bedienen, mit denen der Organismus üblicherweise auf zellschädigende Noxen reagiert.

Bekannterweise wird die Arachidonsäure auf dem Cyclooxygenaseweg nicht nur zu Prostaglandinen, sondern auch zu Thromboxan und Prostacyclin umgewandelt. Unter dem Einfluß von Thromboxan aggregieren die Thrombozyten und haften an der geschädigten Membran an, um diese abzudichten. Um eine Anhaftung zu ermöglichen, werden nicht nur vom Thrombozyt verschiedene Adhäsionsmoleküle präsentiert. Auch das Endothel bereitet sich spezifisch auf die Adhäsion vor. Auf molekularer Ebene werden diese Effekte vor allem über Adhäsionsmoleküle, wie z.B. den von Willebrand Faktor, vermittelt. Die bei der Adhäsion auftretenden Vorgänge spielen sich zeitgleich abgestimmt im Thrombozyten und im Gefäßendothel ab. Es sind vorrangig zwei Abkömmlinge der Arachidonsäurekaskade, welche als Gegenspieler beim Prozess der Blutgerinnung eine entscheidende Rolle spielen: Neben dem Thromboxan, welches die Thrombozytenaggregation bewirkt, ist es der "Gegenspieler" Prostacyclin, welches verhindert, dass sich das reaktive Geschehen über Gebühr ausbreitet. Die Biosynthese von Thromboxan und Prostacyclin wird durch unterschiedliche Enzyme vermittelt.

Thromboxan wird über Cyclooxygenase-1 (COX1), Prostacyclin über Cyclooxygenase-2 (COX2) synthetisiert.

Ein wesentliches Element der Pilzinfektion besteht darin, dass die Pilz-Wirt-Interaktion eine Reihe von mechanistischen Analogien zur Thrombozyten-Endothel-Interaktion aufweist. Dies bedeutet vor allem, dass in den Wirtszellen entweder vorausgehend oder zeitgleich mit der Pilzinfektion, also der Transformation vom apathogenen bzw. fakultativ pathogenen zum pathogenen Erreger, inflammatorische Prozesse ablaufen (z.B. Filler et al., Inf.Immun. 64 (1996), 2609-2617; Cannom et al., J.Inf.Dis. 186 (2002), 389-396), welche über die Arachdinosäurekaskade gesteuert sind, bzw. dass die Adhäsion des Pilzes erst durch vorausgehende inflammatorische Prozesse in den Wirtszellen überhaupt ermöglicht wird. Die Anheftung der Pilze an die Wirtszellen erfolgt von der Pilzseite her über die Hyphen, von Seiten der Wirtszellen im Wesentlich über einen Arachidonsäure abhängigen Mechanismus unter Zunutzemachung der unter diesen Bedingungen an der Wirtszelle exprimierten Adhäsionsmoleküle, wie z. B. dem von Willebrand-Faktor, Vitronektin, Fibronektin oder verschiedenene Integrinen.

Das Vorsehen eines Epithelzellen- oder Endothelzellen-Adhäsionsinhibitors im Kombinationspräparat gemäß der vorliegenden Erfindung ist daher ein wesentliches Element dafür, dass das Antimykotikum im infizierten Bereich bei den erfindungsgemäß zu behandelnden Candia-Mykosen überhaupt zur Wirkung kommt. Die Natur des Epithelzellen- oder Endothelzellen-Adhäsionsinhibitors ist eigentlich nicht kritisch, die Auswahl richtet sich generell nach galenischen Gesichtpunkten für eine (zur Förderung einer) optimale(n) Wirkung des antimykotischen Wirkstoffes. Antimykotischer Wirkstoff und Epithelzellen- oder Endothelzellen-Adhäsionsinhibitor werden daher auch aufgrund ihrer physikochemischen Eigenschaften im Kombinationspräparat optimiert. Dieser erfindungsgemäße Adhäsionsinhibitor übt einen Einfluss auf den Arachidonsäure-Metabolisumus aus, so dass die erfindungsgemäße Kombination synergistisch auf die Pilzinfektion an der Infektionsstelle und unter Ausnützung der Patienten-eigenen Arachidonsäure-Mechanismen wirken können.

Bevorzugterweise wird daher der Epithelzellen- oder Endothelzellen-Adhäsionsinhibitor ausgewählt aus
- einem nicht-steroidalen Antiphlogistikum (NSAID) mit einer ausreichenden COX1-Hemmung, welche sich einerseits aus der intrinsischen Aktivität, andererseits aus dem Ausmaß der Selektivität der Hemmung von COX1 gegenüber COX2 unter therapeutischen Bedingungen ergibt. Dies bedeutet, dass alle Substanzen, welche eine selektivere COX2-Hemmung als z.B. Meloxicam oder Diclofenac zeigen, im Sinne der Erfindung ungeeignet sind (wie selbstverständlich auch alle Substanzen, die keine Aktivität bzw. Auswirkungen auf den Arachidonsäure-Metabolismus zeigen). Die zahlenmäßige Festlegung erfolgt beispielsweise durch die Angabe der COX1/COX2-Ratio der IC50- Werte, diese differiert jedoch mit der verwendeten Methode. Geeignete Verbindungen können zum Beispiel entsprechend den Angaben von K. Brune et al. (Deutsches Ärzteblatt 97, (26) 2000, A-1818/B-1538/C-1434) zahlenmäßig durch ein COX1/COX2-Verhältnis (IC50) von ≤ 20, definiert werden.

NSAIDs sind gegenwärtig bereits arzneimittelrechtlich zugelassen.

Es sind daher Arzneimittel im erfindungsgemäßen Kombinationspräparat enthalten, welche die Thrombozytenaggregation hemmen, wie vor allem NSAID mit COX1-Hemmung, bzw. Arzneimittel, welche die Expression oder Funktion von mit diesem Vorgang verbundenen Adhäsionsmolekülen im Endothel unterbinden, in analoger Weise die Adhäsion von Erregern am Endothel bzw. Epithel erschweren oder verhindern. Dies geschieht nicht nur durch die Unterdrückung der Expression geeigneter Adhäsionsmoleküle, sondern vor allem auch durch die Auslösung/Verstärkung des "shedding" dieser Oberflächenmoleküle. In gleicher Weise können Prostacyclin selbst und Prostacyclinabkömmlinge die Adhäsion reduzieren bzw. unterbinden. Der erfindungsgemäße Einsatz von derartigen Verbindungen in Kombination mit einem Antimykotikum verhindert somit sowohl die Infektion, als auch das - durch inflammatorische Vorgänge im Zuge der Infektion ausgelöste - "Aufschaukeln" der Infektion. Die Erkenntnis, dass sich ein so komplexer Prozess, wie die Adhäsion eines Pilzes an das Endothel des Wirtes, durch Wirkstoffe einer altbekannten Klasse beeinflussen lässt, ist bisher unbekannt und bildet die Grundlage der erfindungsgemäßen Kombinationspräparate, mit welchen die überraschenden klinischen Ergebnisse gemäß der vorliegenden Erfindung erzielt werden konnten, insbesondere bei den beschriebenen bevorzugten Candida-Mykosen, die bislang nicht oder nur schwer behandelbar waren.

Die erfindungsgemäßen Arzneistoffkombinationen sind zur topischen Anwendung (an Schleimhäuten bzw. auch an stark entzündeten Hautarealen, wie beispielsweise beim Windelsoor oder beim intertriginösen Ekzem) zur Behandlung von Pilzerkrankungen (Vulvovaginitis, oropharyngeale Candidiasis (Mundsoor), Windeldermatitis, intertriginöses Ekzem), bestimmt und bestehen wie erwähnt aus einem Antimykotikum und einem Epithelzellen- oder Endothelzellen-Adhäsionsinhibitor, welcher durch Unterdrückung der oben beschriebenen Mechanismen die Anhaftung der Erreger an Epithel- und/oder Endothelzellen und die Bildung einer schichtförmigen Besiedelung des betroffenen Epithel- bzw. Endothelbezirks erschwert bzw. verhindert.

Besonders bevorzugt ist dabei, wie oben erwähnt, die Verwendung einer NSAID-Verbindung. Jedoch sind dabei keineswegs alle NSAID geeignete Verbindungen im Kombinationspräparat gemäß der vorliegenden Erfindung, da NSAIDs allgemein je nach ihrem Profil (COX1-Hemmer oder COX2-Hemmer) auf die Adhäsion entweder einen fördernden (COX2) oder hemmenden Effekt (COX1) ausüben. Aus diesem Grund ist die Behandlung dermatologischer Erkrankungen mit einer Kombination, welche einen selektiven COX2-Hemmer enthält (wie beispielsweise in der US 2005/0014729 A geoffenbart) in Bezug auf die therapeutische Zielsetzung der vorliegenden Erfindung, nämlich die Reduktion der Adhäsion im Endothel bzw. Epithel des Patienten herbeizuführen, verfehlt. Hochselektive COX2-Hemmer oder anti-inflammatorische Mittel ohne Aktivität auf den Arachidonsäure-Mechanismus (wie z.B. Benzydamin (Riboldi et al., Br.J.Pharmacol. 140 (2003), 377-383)) sind keine relevanten Verbindungen im Sinne der vorliegenden Erfindung.

Die Selektivität der NSAID wird z.B. durch die Ratio der IC 50 (microM) COX1/COX2 ausgedrückt, die Werte sind jedoch im Allgemeinen abhängig von den verwendeten pharmakologischen Testsystemen und differieren stark, daher werden erfindungsgemäß die Werte entsprechend den Angaben von K. Brune et al. (Deutsches Ärzteblatt 97, (26) 2000, A-1818/B-1538/C-1434) zahlenmäßig festgelegt.

Wesentlich für die therapeutische Wirksamkeit ist eine ausreichende COX1-Hemmung (Absolutwert der IC 50) bei der jeweils verabreichten therapeutischen Dosis. Die meisten NSAID sind gemischte COX1/COX2-Hemmer. Solange die COX1-Hemmung gegeben ist, ist die COX2-Hemmung durchaus willkommen (wegen der Schmerzunterdrückung). Daher sind NSAID ohne oder mit nur geringer Bevorzugung der COX2 erfindungsgemäß anzuwendende Substanzen. Ungeeignet sind jedoch, wie erwähnt, (hoch)selektive COX2-Hemmer, welche unter therapeutischen Bedingungen (Dosierungen) keine bzw. eine zu geringe COX1-Wirkung entfalten. Die selektiven COX2-Hemmer können, im Gegenteil, das Krankheitsbild sogar negativ beeinflussen und sind daher im Rahmen der erfindungsgemäßen Therapie zu vermeiden. Demgemäß sind erfindungsgemäß NSAIDs, die für das COX1/COX2-Verhältnis einen Wert von > 20 aufweisen (bezogen auf o.g. Lit.), auszuschließen (für zugelassene hochselektive COX2-Hemmer ergeben sich Werte von über 100, z.B. Celecoxib, Rofecoxib, Valdecoxib, Etoricoxib).

Demgemäß ist das NSAID im Kombinationspräparat gemäß der vorliegenden Erfindung vorzugsweise ausgewählt aus Diclofenac, Fenclofenac, Alclofenac, Lonazolac, Clidanac, Oxipinac, Clopinac Tolmetin, Indomethacin, Mefenaminsäure, Flufenaminsäure, Meclofenaminsäure, Tolfenaminsäure, Nifluminsäure, Floctafenin, Bucloxinsäure, Ibuprofen, Dextroprofen, Prapoprofen, Miroprofen, Fenoprofen, Fluprofen, Flurbiprofen, Ketoprofen, Alminoprofen, Tioxaprofen, Tiaprofensäure, Isoxepac, Nimesulid, Meloxicam, Tenoxicam, Lornoxicam, Piroxicam, Droxicam Sudoxicam, Naproxen, Bufexamac, Etofenamat, Felbinac, Nabumeton, Ketorolac, Etodolac, Oxaprocin, Salicylsäure, Acetylsalicylsäure, Flufenisal, Diflunisal, Benorylat, Fentiazac, Azapropazon, Phenylbutazone, Kebuzon, pharmazeutisch wirksame Salze oder Ester dieser Substanzen oder Mischungen dieser Substanzen, insbesondere Diclofenac, Nimesulid, Fenclofenac, Alclofenac, Lonazolac, Tolmetin, Indometacin, Mefenaminsäure, Flufenaminsäure, Meclofenaminsäure, Floctafenin, Ibuprofen, Flurbiprofen, Ketoprofen, Alminoprofen, Meloxicam, Tenoxicam, Lornoxicam, Naproxen, Etofenamat, Felbinac, Nabumeton, Ketorolac oder Etodolac.

Der besondere Stellenwert der erfindungsgemäßen Kombination von Antimykotikum und Epithelzellen- oder Endothelzellen-Adhäsionsinhibitor hängt unmittelbar vom Ausmaß des Effektes der zweiten Komponente ab, welche primär durch eine die Adhäsion unterdrückende Wirkung auf das Endothel bzw. Epithel des Patienten zum therapeutischen Effekt beiträgt. Daher ist es von besonderer Bedeutung zu berücksichtigen, dass der Begriff "Hauterkrankungen" einen breiten Bereich völlig unterschiedlich aufgebauten Hautgewebes erfaßt: Vom Nagel eines Erwachsenen bis zur Schleimhaut eines Kleinkindes. Allen Erkrankungen, die als bevorzugte Indikation für das Kombinationspräparat gemäß der vorliegenden Erfindung genannt sind (vulvovaginale Candidiasis, oropharyngeale Candidiasis (Mundsoor), Windeldermatitis (Windelsoor) und intertriginöses Ekzem), ist gemeinsam, dass die Oberfläche der betroffenen Gewebsbezirke eine Anhaftung der Pilzkolonien aufgrund der Expression entsprechender Haftmoleküle erst ermöglicht. Das erfindungsgemäße Kombinationspräparat dient zur Therapie von Pilzerkrankungen, vorzugsweise zur Therapie von Candidamykosen, welche typischerweise an den genannten Hauttypen, also Schleimhäuten des Urogenitaltraktes und des Oropharyngealbereiches, sowie an vorgeschädigter (mazerierter, verletzter, entzündlich veränderter) Außenhaut auftreten.

Demgemäß ist es nicht Gegenstand der vorliegenden Erfindung, mit dem beschriebenen Kombinationspräparat eine Therapie von Dermatophyten-Erkrankungen durchzuführen (EP-1 390 031-B1), welche häufig die Hornhautschichten der Epidermis besiedeln, da hier der Beitrag zum therapeutischen Erfolg, welcher durch die Unterbindung der Adhäsion des Pilzes an das Wirtsepithel geleistet wird, deutlich geringer ist und daher der erfindungsgemäße Effekt nicht eintritt. Dies gilt auch z.B für die Behandlung von Nagelmykosen (WO 2000/028821 A1), auch hier ist der Einsatz des erfindungsgemäßen Kombinationspräparates nicht zielführend, da auch hier der überraschende Synergismus der zwei Komponenten im erfindungsgemäßen Präparat nicht (im erfindungsgemäßen Ausmaß) auftritt, insbesondere, weil in diesem Fall der Zustand des Epithels im Vergleich zu den Besonderheiten der Therapie dieser Erkrankungen, insbesondere der schlechten "Zugängigkeit" des Therapeutikums, von untergeordneter Bedeutung ist.

Bei der erfindungsgemäßen topischen Therapie mit dem beschriebenen Kombinationsarzneimittel an Schleimhäuten bzw. vorgeschädigter Außenhaut sind im Vergleich zu Erkrankungen der normalen bzw. stärker verhornten Außenhaut und ihrer Anhangsgebilde erfindungsgemäß nicht nur grundsätzlich bedeutend niedrigere Konzentrationen an Wirkstoff ausreichend, es kommt auch innerhalb wesentlich kürzerer Zeit (Minuten bis wenige Stunden vs. Tage bis Wochen unter Anwendung der derzeit üblichen Behandlungsmethoden) zu einer signifikanten Verbesserung der subjektiven und objektiven Erkrankungssymptome. Somit ist die besonders niedrige Dosierung des adhäsionshemmenden Wirkstoffes ein wesentlicher Vorteil bei der vorliegenden Erfindung. Dieser schnelle Wirkungseintritt ist allein durch die Vorgänge an der entzündlich veränderten Haut- bzw. Schleimhautoberfläche hervorgerufen und daher weder durch in-vitro-Tests noch durch Untersuchungen an der nicht entzündeten Außenhaut vorhersagbar oder prinzipiell feststellbar. Da die massiv verstärkte Wirkung allein auf der Basis von Mechanismen an der exponierten Oberfläche des jeweilig betroffenen Organs zustande kommt (Expression und Abstoßung der Anhaftungsstellen), ist speziell auch dieser Effekt gerade durch in-vitro-Experimente nicht bestimmbar, es hat sich auch tatsächlich gezeigt, dass in-vitro-Experimente, abhängig vom verwendeten Pilzstamm, von der Dosierung und den verwendeten Arzneistoffkombinationen widersprüchlichste Ergebnisse lieferten. Bemerkenswert in diesem Zusammenhang ist auch, dass die Kombination von Fluconazol mit Ibuprofen bei Fluconazol-sensiblen Stämmen im in-vitro-Experiment keinerlei synergistischen Effekt zeigte (Arai et al., 2005). Der Effekt, der mit dem erfindungsgemäßen Kombinationspräparat erzielbar ist, kann daher auch nicht auf diesem Weg (über in-vitro-Experimente) abgeleitet werden. Auch bei systemischer Verabreichung des erfindungsgemäßen Kombinationspräparates, insbesondere des Epithelzellen- oder Endothelzellen-Adhäsionsinhibitors (also z.B. des erfindungsgemäß ausgewählten NSAID) ist aus pharmakokinetischen Gründen ein entsprechender Effekt nicht einmal bei üblicher Dosierung zu erwarten, erst recht nicht bei den niedrigen Dosierungen, welche mit der vorliegenden Erfindung ermöglicht werden.

Die niedrige erforderliche Dosis ist auch dadurch bedingt, dass der Epithelzellen- oder Endothelzellen-Adhäsionsinhibitor sowohl am Pilz als auch im Epithel des Patienten eine Wirkung ausübt.

Damit ist auch eine Behandlung von Säuglingen und Schwangeren möglich.

Die Wirkung der erfindungsgemäßen Arzneistoffkombination beruht somit
1. auf der Unterdrückung des Erregerwachstums,
2. auf der Unterdrückung der Adhäsion des Erregers an der Wirtszelle,
3. auf der Unterdrückung der akuten Entzündungs- und Schmerzsymptomatik durch Hemmung der Prostaglandinsynthese auf der Seite des betroffenen Organismus,
4. auf der Verhinderung der Entstehung der begünstigenden Faktoren
   im lokalen Environment, nämlich der Adhäsion an der Wirtszelle nach Transformation des Erregers und damit in der Verhinderung der Aggravierung des Krankheitsbildes und
5. auf dem Durchbrechen der pathogenetischen Mechanismen bei erfolgter Aggravierung/Chronifizierung,
wobei 2.-5. durch den Epithelzellen- oder Endothelzellen-Adhäsionsinhibitor (welcher in die Arachidonsäurekaskade eingreift) und 1. entsprechend dem Wirkungsmechanismus des verwendeten Antimykotikums vermittelt wird.

Dies ermöglicht ein gegenüber dem Stand der Technik völlig neuartiges Therapiekonzept, welches sich - neben der alleine schon überraschenden Wirksamkeit bei (chronisch- rezidivierender) vulvovaginaler Candidiasis, oropharyngealer Candidiasis (Mundsoor), Windeldermatitis (Windelsoor) und intertriginösem Ekzem - noch durch die folgenden Vorteile und Effekte gegenüber dem Stand der Technik auszeichnet:
1. Möglichkeit der Lokaltherapie,
2. Bei Chronifizierung deutliche Reduktion der Rezidivneigung,
3. Stark beschleunigter Wirkungseintritt,
4. Signifikant verringerte Dosierung des Antimykotikums und
5. Sofortige Schmerzstillung

Ad 1. Bei begünstigenden Faktoren, insbesondere auch für eine Aggravierung/Chronifizierung, z.B. Immunsuppression, hormonelle und genetische Faktoren, wird gemäß der vorliegenden Erfindung der pathogenetische Mechanismus, auf dem die Erkrankungsdisposition beruht, gehemmt. Die Krankheit kann ohne systemische Therapie kontrolliert bzw. zur Ausheilung gebracht werden. Die Vermeidung des Übergangs auf systemische Therapie (die oft auch unter Anwendung stärker belastender Arzneistoffe erfolgt) ist sowohl im Fall einer bestehenden Schwangerschaft als auch bei Patienten mit HIV, Leukämie oder unter Chemotherapie von Bedeutung und bringt eine deutliche Verringerung der Belastung des Patienten, sowohl nach subjektiven (Compliance) als auch nach objektiven (Stoffwechselbelastungs-) Kriterien. Die chronisch-rezidivierende Vulvovaginitis in der Schwangerschaft ist mit dem herkömmlichen Therapieschema kaum unter Kontrolle zu bringen, da die systemische Anwendung der oral applizierbaren Antimykotika (Fluconazol) in der Schwangerschaft absolut kontraindiziert ist.

Ad 2. Auch bei chronisch-rezidivierendem Verlauf (insbesonders bei Vulvovaginitis durch Candida-Infektionen) wird der zugrundeliegende pathogenetische Mechanismus unterbrochen und statt der langwährenden systemischen Therapie eine topische Therapie ermöglicht.

Ad 3. Gegenüber der bisher üblichen Therapie erfolgt der Wikungseintritt stark beschleunigt, das heißt innerhalb von Minuten (Vulvovaginitis) bis Stunden (Windelsoor) gegenüber Tagen bis Wochen (bis überhaupt nicht) bei herkömmlicher Therapie.

Der Unterschied liegt grundsätzlich v.a. in der Behandlungsdauer, das eigentliche Therapieschema hängt dabei allerdings in erster Linie von der Halbwertszeit der verwendeten Arzneistoffe ab. Beispielsweise kann der Unterschied zuhand der chronisch-rezidivierenden Vulvovaginitis deutlich gemacht werden:
Erfindungsgemäße Anwendung:
Initialtherapie: 3-5 x tgl. lokale Applikation der Arzneistoffkombination als Salbe oder mittels einer anderen lokal applizierbaren Arzneiform über drei bis fünf Tage;
bei erneut auftretenden Beschwerden 2-3 x tgl. lokale Applikation über einen Tag ausreichend (die rezidivfreien Intervalle werden mit fortdauernder Anwendung immer länger).

Im Gegensatz dazu das derzeitige Therapieschema (Quelle: Leitlinien der deutschen Gesellschaft für Gynäkologie und Geburtshilfe) :
Initialtherapie:
Fluconazol 150 mg oral 1 oder 2 x/Woche über 4-6 Wochen anschließend
Fluconazol 150 mg oral 1 x/2 Wochen für 2-3 Monate anschließend
Fluconazol 150 mg oral 1 x/4 Wochen über 4-6 Monate Nach dem Absetzen rezidivieren ca. 50% der Fälle wie vor der Therapie.

Ad 4: Aufgrund der potenzierten Wirkung kann die Gesamtdosis des Antimykotikums deutlich reduziert werden, im Fall der unkomplizierten Vulvovaginalmykose kann man aufgrund der reduzierten Behandlungsdauer von einer um mindestens 50% reduzierten Gesamtdosis ausgehen, bei der oropharyngealen Mykose kann die Reduktion bis zu 66% betragen.

Ad 5: Bei den oft überaus schmerzhaften akuten Krankheitsbildern wird durch die gleichzeitige topische Anwendung von NSAID mit dem Antimykotikum eine sofortige Schmerzstillung und Abschwellung bei fehlender bis minimaler Gesamtbelastung des Organismus mit den systemisch nicht nebenwirkungsfreien NSAID (nicht steroidalen Antiphlogistika) erreicht. Wie erwähnt, ist die Basis der erfindungsgemäßen Behandlung der Befund, dass die Expression bestimmter Adhäsionsmoleküle an der Gewebsoberfläche des erkrankten Organismus bzw. Organs eine grundlegende Voraussetzung für die Kolonialisierung durch Hefepilze darstellt. Die Kombination herkömmlicher Antimykotika mit Epithelzellen- oder Endothelzellen-Adhäsionsinhibitoren greift daher unmittelbar in den pathogenetischen Prozess ein. Epithelzellen- oder Endothelzellen-Adhäsionsinhibitoren sind, wie erwähnt, Substanzen, welche die Anhaftung der Erreger an Epithel- und/oder Endothelzellen und die Bildung einer schichtförmigen Besiedelung des betroffenen Epithel/bzw. Endothelbezirks erschweren bzw. verhindern. Aus der Natur der verantwortlichen Adhäsionsmoleküle ergibt sich, dass deren Expression durch Wirkstoffe, die auf bestimmte Art in den Prostaglandinstoffwechsel eingreifen, unterbunden wird. Durch die erfindungsgemäße kombinierte Anwendung von antimykotisch wirksamen Substanzen und Epithelzellen- oder Endothelzellen-Adhäsionsinhibitoren ergibt sich eine Potenzierung des therapeutischen Effektes. Die Behandlungsdauer wird stark verkürzt und eine lokale Behandlung wird auch in Fällen möglich, bei denen bisher ausschließlich eine systemische Therapie Aussicht auf Erfolg hatte. Darüber hinaus wirkt diese Art einer Arzneistoffkombination auch in Fällen, die gegenüber den derzeit verfügbaren Arzneimitteln völlig therapieresistent sind.

Gegenstand der Erfindung ist daher die Verwendung der erfindungsgemäßen Arzneistoffkombinationen für die topische Anwendung an Schleimhäuten und an stark entzündeten bzw. mazerierten Hautarealen zur Behandlung von Pilzerkrankungen, bevorzugt solche, welche durch Candida spp. hervorgerufen werden (Vulvovaginitis, oropharyngeale Candidiasis (Mundsoor), Windeldermatitis (Windelsoor), intertriginöses Ekzem).

Die Applikation des erfindungsgemäßen Kombinationspräparates erfolgt ausschließlich lokal (Salben, Vaginaltabletten, -zäpfchen usw.) an Haut und Schleimhäuten. Die Dosierung des Antimykotikums (vorzugsweise Clotrimazol) erfolgt in der bisher üblichen Konzentration, allerdings ergibt sich eine Halbierung der täglichen Gesamtdosis und eine deutliche Verkürzung der Gesamtbehandlungsdauer (von 7 auf 2-3 Tage). Der Epithelzellen- oder Endothelzellen-Adhäsionsinhibitor wird in 1/5 bis 1/100 der täglichen Maximaldosis als Beimengung zur Salbe/Vaginaltablette etc. verabreicht.

Bei der topischen Therapie mit einer erfindungsgemäßen Arzneistoffkombination sind wie oben erwähnt im Vergleich zur bisherigen antimykotischen Monotherapie bedeutend niedrigere Wirkstoffgesamtmengen ausreichend, auch die NSAID-Komponente wird in einer signifikant niedrigeren Konzentration eingesetzt als in allen anderen Indikationen, für die bisher Arzneimittel aus solchen Stoffen in Verwendung sind. Damit ist auch eine Behandlung von Säuglingen und Schwangeren möglich.

Es kommt innerhalb wesentlich kürzerer Zeit (Minuten bis wenige Stunden gegenüber Tagen bis Wochen bei Anwendung der derzeit üblichen Behandlungsmethoden) zu einer signifkanten Verbesserung der subjektiven und objektiven Erkrankungssymptome. Dieser schnelle Wirkungseintritt ist allein durch die Vorgänge an der Schleimhautoberfläche hervorgerufen und ursächlich auf die Wirkung von Arachidonsäureabkömmlingen ausgelösten Wirkungen an der betroffenen Organoberfläche zurückzuführen und daher weder durch in-vitro-Tests noch durch Untersuchungen an normaler Außenhaut feststellbar.

Die Natur des im vorliegenden Kombinationspräparat einzusetzenden antimykotischen Wirkstoffs ist prinzipiell nicht kritisch, in der Regel wird immer ein optimiertes Kombinationspaar eingesetzt, wobei sich die Optimierung vor allem auf das antimykotische Spektrum des Anti-Mykotikums, auf die Galenik und auf die physikochemische Wechselwirkungen des Antimykotikums mit dem Epithelzellen- oder Endothelzellen-Adhäsionsinhibitor bezieht.

Bevorzugt sind natürlich diejenigen antimykotischen Wirkstoffe, für die bereits eine arzneimittelrechtliche Zulassung besteht.

Daher ist der antimykotische Wirkstoff im erfindungsgemäßen Kombinationspräparat vorzugsweise ein Azol oder Konazol, vorzugsweise Clotrimazol, Bifonazol, Croconazol, Miconazol, Econazol, Isokonazol, Itraconazol, Fenticonazol, Tioconazol, Sertaconazol, Sulconazol, Omoconazol, Oxiconazol, Fluconazol, Voriconazol oder Ketokonazol, insbesondere Clotrimazol und Miconazol, ein Squalenepoxidasehemmer, vorzugsweise Naftifin oder Terbinafin, oder ein Antibiotikum, vorzugsweise Nystatin, Amphotericin B, Capsofungin oder Natamycin,
oder Tolciclat, Tolnaftat, Ciclopirox, Haloprogin, Butenafine, Flucytosin.

Vorzugsweise ist das Kombinationsarzneimittel gemäß der vorliegenden Erfindung als Salbe, Creme, Lotion, Gel, Tinktur, Lösung, Vaginal-Suppositorium, Vaginal-, Buccal- oder Sublingual-Tablette, Sirup, Suspension, Puder, Spray oder Aerosol hergestellt.

Gemäß einer bevorzugten Ausführungsform der vorliegenden Erfindung kann das Kombinationsarzneimittel an einem inerten Träger vorgesehen werden, insbesondere an einem Vaginalring, einem Diaphragma oder einem Tampon.

Die Erfindung wird anhand der nachfolgenden Beispiele näher beschrieben, ohne darauf eingeschränkt zu sein.

### Untersuchungen und Ergebnisse am Menschen - Fallbeispiele

Das erfindungsgemäße Therapiekonzept wurde im Rahmen der ärztlichen Verschreibungsfreiheit bisher an freiwilligen Probanden untersucht. Bei allen Patienten war eine erfolglose antimykotische Monotherapie der erfindungsgemäßen Kombinationstherapie vorausgegangen.

Im Folgenden werden 6 Beispiele beschrieben:
Vulvovaginitis: 3 Fälle
Windelsoor: 2 Fälle
Mundsoor: 1 Fall

Die Behandlung erfolgte durch die topische Administration von geeigneten Arzneistoffkombinationen auf der Haut bzw. den Schleimhäuten des Urogenitaltraktes und des Oropharynx.

Folgende Kombinationen wurden verwendet:
Vulvovaginitis:
Fall 1:
41jährige Patientin, chronisch-rezidivierende Candida-Vulvovaginitis seit Jahren (> 10 Rezidive/Jahr), ansonsten gesund, gravid, Vorbehandlung (vor der Schwangerschaft) sowohl topisch (Clotrimazol, Nystatin) als auch systemisch (Fluconazol, mehrfach, auch Dauertherapie und Partnertherapie). Massive Verschlechterung mit völliger Therapieresistenz gegen Lokalbehandlung mit Clotrimazol in der Schwangerschaft, extreme subjektive Beschwerden seit Wochen.

Gyn. Befund: Vulva stark gerötet, deutlich geschwollen, blutige Excoriationen. Vaginal- und Portioschleimhaut gerötet. Massiver Fluor vag., Abstrichbefund: PAP 2., Mikrobiol.Abstrich, Nativbefund: reichlich Leukozyten, massiv Pilzhyphen nachweisbar, RG 3.

Therapie mit einer Salbenkombination von Clotrimazol/Diclofenac-Na

Einzeldosis: 5 mg Diclofenac-Na/20 mg Clotrimazol Dosierungsschema: Initial (3 Tage) 3 x täglich Salbenstreifen von 2.5 cm lokal in Vulva und Vagina appliziert, anschließend einmal tgl. über 4 Tage.

Die applizierte Dosis (Gesamtdosis) an Diclofenac-Na beträgt ca. 1/30 der maximalen Tagesdosis bei systemischer Applikation. Die Clotrimazoldosis beträgt die Hälfte der üblichen Tagesdosis.

Befund nach einwöchiger Therapie (drei Tage nach Beendigung der Therapie):
Pat. subjektiv beschwerdefrei (seit Therapiebeginn). Gyn. Befund: Vulva o.B. Vaginal- und Portioschleimhaut o.B. Geringgradiger Fluor vag. Mikrobiol.Abstrich, Nativbefund: Keine Pilzhyphen nachweisbar, vereinzelt Sporen. Normale Scheidenflora (Lactobacillen), RG 1.

Follow-up: 6 Monate
Im Verlauf der nächsten drei Monate ca. 2 Rezidive/Monat, eintägige Behandlung wie oben brachte jeweils sofortige Beschwerdefreiheit.

Folgende Monate: völlige Beschwerdefreiheit, keine neuerlichen Rezidive.

### Fall 2:

43jährige Patientin, chronisch-rezidivierende Candida-Vulvovaginitis seit Jahren (> 10 Rezidive/Jahr), ansonsten gesund, Vorbehandlung sowohl topisch (Clotrimazol, Miconazol) als auch systemisch (Fluconazol, mehrfach, auch Dauertherapie und Partnertherapie). Akute Exacerbation, geringgradige Verbesserung unter Therapie mit Clotrimazol (8 Tage), teils extreme subjektive Beschwerden über 2 Wochen.

Gyn. Befund: Vulva stark gerötet, deutlich geschwollen. Vaginal- und Portioschleimhaut gerötet. Massiver Fluor vag.

Initialtherapie: Clotrimazol/Diclofenac (25/25 mg) supp. über 2 Tage, anschließend

Clotrimazol/Diclofenac als Salbe wie unter Fall 1 beschrieben über 3 Tage.

Befund nach 1 Woche: Pat. subjektiv beschwerdefrei (seit 2.Tag nach Therapiebeginn). Gyn. Befund: Vulva o.B. Vaginal- und Portioschleimhaut o.B. Geringgradiger Fluor vag.

### Fall 3:

42jährige Patientin, chronisch-rezidivierende Candida-Vulvovaginitis seit Jahren (> 10 Rezidive/Jahr), ansonsten gesund, Vorbehandlung sowohl topisch (Clotrimazol, Nystatin) als auch systemisch (Fluconazol, mehrfach, auch Dauertherapie und Partnertherapie). Akute Exacerbation, während dieses Rezidivs noch keine Vorbehandlung, mäßige subjektive Beschwerden seit 2 Tagen.

Gyn. Befund: Vulva gerötet, geschwollen. Vaginal- und Portioschleimhaut gerötet. Verstärkter Fluor vag.

Therapie: Clotrimazol/Diclofenac als Salbe wie unter Fall 1 beschrieben am ersten Tag 5 x täglich, ab 2. Tag 3 x tgl. über insgesamt drei Tage.

Befund nach 1 Woche: Pat. subjektiv beschwerdefrei (seit 18 Stunden nach Therapiebeginn). Gyn. Befund: Vulva o.B. Vaginal- und Portioschleimhaut o.B. Geringgradiger Fluor vag.

### Fall 4:

### Windelsoor:

Weibl. Säugling, 12 Monate, über 5 Wochen therapieresistentes Ekzem an den äußeren großen Schamlippen, (Vorbehandlung durch Pädiater und Dermatologen mit: Zinkoxid-hältiger Babycreme, Nystatin-hältiger Creme, antibiotischer (antibakterieller) Creme und Puder, Clotrimazol-hältiger Creme, Corticosteroiden. Unter diesen Therapien kontinuierliche Verschlechterung des klinischen Bildes.

Insp.: Ausgeprägte Rötung und Schwellung der äußeren Schamlippen und perianal.

Therapie mit einer Salbenkombination von Clotrimazol/Diclofenac-Na

Einzeldosis: Salbenstreifen von ca. 5 cm abends, (entspricht ca. 10 mg Diclofenac-Na /40 mg Clotrimazol), totale Abheilung über Nacht. Anschließend Darmsanierung mit Nystatin oraler Suspension.

Follow-up (6 Monate): keine weiteren Beschwerden

Fall 5: Windelsoor: Weibl. Säugling, 2 Monate, wiederholt Windelsoor (rote Papeln).

Insp.: Kleine Papeln an der Haut der großen Schamlippen und in der Perianalregion.

Therapieversuch: Während verschiedener Episoden Vergleich von Clotrimazolcreme mit und ohne Zugabe von Diclofenac-Na. Monotherapie: Clotrimazolcreme (Canesten), Einzeldosis ca. 25 mg Clotrimazol

Kombination: Clotrimazol/Diclofenac-Na, Einzeldosis: ca. 5 mg Diclofenac-Na / 20 mg Clotrimazol

In beiden Fällen lokale Applikation, Dosierung: 4-5 x Tag

### Ergebnis:

Monotherapie: Behandlungsdauer bis zum völligen Verschwinden der Läsionen: 3 Tage

Kombinationstherapie: Behandlungsdauer bis zum völligen Verschwinden der Läsionen: 24 Stunden

### Fall 6: Mundsoor:

Weibl. Säugling, 2 Monate, wiederholt Mundsoor (weiße Läsionen). Insp.: Typische weiße Beläge an der Innenseite der Ober- und Unterlippe, Durchmesser 4-5 mm

Therapieversuch: Während verschiedener Episoden Vergleich von Miconazolgel mit und ohne Zugabe von Mefenaminsäure. Monotherapie: Miconazol- (Daktarin-Gel), Einzeldosis 30 mg Kombination: Miconazol/Mefenaminsäure (Gel)

In beiden Fällen lokale Applikation, Dosierung: 2-3 x Tag Einzeldosis: ca. 25 mg Mefenaminsäure/30 mg Miconazol

### Ergebnis:

Monotherapie: Behandlungsdauer bis zum völligen Verschwinden der Läsionen: 5 Tage

Kombinationstherapie: Nach 12 Stunden signifikante Reduktion der Läsionen, Behandlungsdauer bis zum völligen Verschwinden der Läsionen: 24 Stunden

### Zusammenfassung der Ergebnisse der Einzelfallstudien:

Vulvovaginitis: Bei den beschriebenen Fällen hatte es sich um besonders schwere Fälle von chronisch-rezidivierender Candidavaginitis gehandelt. Unter Monotherapie mit Clotrimazol war es im zweiten Fall zu einem stark verzögerten Heilungsverlauf gekommen (8 Tage Intensivtherapie, danach Besserung, aber keine Beschwerdefreiheit), im ersten und dritten Fall ließ sich keine Verbesserung durch herkömmliche lokale Therapie mit Clotrimazol erzielen. Durch den Einsatz der Arzneistoffkombinationen kam es zu einer sofortigen deutlichen Verbesserung der subjektiven und objektiven Symptome und schon am zweiten Behandlungstag zu einem vollständigen Verschwinden der Beschwerden in allen Fällen. Windelsoor: Unter Clotrimazol-Monotherapie deutliche Verschlechterung des Krankheitsbildes im ersten Fall, Abheilung nach drei Tagen im zweiten (leichten) Fall. Vollständige Abheilung nach Einsatz der Arzneistoffkombination innerhalb von 12 bzw. 24 Stunden.

Mundsoor: Abheilung mit Miconazol-Monotherapie nach 4-5 Tagen (diagnostisches Kriterium: keine Plaques mehr in der Mundhöhle sichtbar). Im Rahmen einer zweiten Episode Behandlung mit der Arzneistoffkombination, nach zwei Stunden keine Plaques mehr sichtbar.

Diese Ergebnisse zeigen, dass das erfindungsgemäße Kombinationspräparat in besonders schwierig zu behandelnden Candidamykosen eine überraschend schnelle und umfassende Heilung bringt, die mit der Monotherapie (Antimykotikum allein) nicht erreicht werden konnte.

Der Behandlungserfolg war umso deutlicher, je ausgeprägter der Anfangsbefund war.

## Patentansprüche

1. : Verwendung von
- einem antimykotischen Wirkstoff und
- einem Epithelzellen- oder Endothelzellen-Adhäsionsinhibitor zur Herstellung eines Kombinationsarzneimittels zur topischen Behandlung von Candidamykosen ausgewählt aus vulvovaginaler Candidiasis, oropharyngealer Candidiasis (Mundsoor), Windeldermatitis (Windelsoor) und intertriginösem Ekzem,
wobei der Epithelzellen- oder Endothelzellen-Adhäsionsinhibitor ausgewählt ist aus einem nicht-steroidalen Antiphlogistikum (NSAID) mit einer ausreichenden COX1-Hemmung, definiert durch den Ausschluss von Verbindungen mit einem COX1/COX-2-Verhältnis (IC50, microM) von > 20, wobei das Kombinationsarzneimittel eine Salbe, eine Creme, ein Vaginal-Suppositorium, eine Vaginal-, Buccal- oder Sublingual-Tablette, ein Puder, oder ein Aerosol ist.

2. : Verwendung nach Anspruch 1, **dadurch gekennzeichnet, dass** der antimykotische Wirkstoff ein Azol oder Konazol, vorzugsweise Clotrimazol, Bifonazol, Miconazol, Econazol, Isokonazol, Itraconazol, Fenticonazol, Tioconazol, Serticonazol, Omoconazol, Oxiconazol, Fluconazol, insbesondere Clotrimazol, ein Squalenepoxidasehemmer,
vorzugsweise Naftifin oder Terbinafin, oder
ein Polyenantimykotikum, vorzugsweise Nystatin, Amphotericin B, Capsofungin oder Natamycin,
oder, Ciclopirox, Butenafine, Flucytosin ist.

3. : Verwendung nach Anspruch 1 oder 2, **dadurch gekennzeichnet, dass** das NSAID ausgewählt ist aus Indomethacin, Mefenaminsäure, Ketoprofen, Acetlysalicylsäure, Ibuprofen, Lornoxicam, Flufenaminsäure, Diclofenac, Piroxicam, Bufexamac, Etofenamat, Felbinac, Tenoxicam, pharmazeutisch wirksame Salze oder Ester dieser Substanzen oder Mischungen dieser Substanzen, insbesondere Diclofenac, Ibuprofen oder Lornoxicam.

4. : Verwendung nach einem der Ansprüche 1 bis 3, **dadurch gekennzeichnet, dass** das Kombinationsarzneimittel an einem inerten Träger vorgesehen ist, insbesondere an einem Vaginalring, einem Diaphragma oder einem Tampon.

## Claims

1. : Use of
- an antifungal active ingredient and
- an epithelial-cell or endothelial-cell adhesion inhibitor for producing a combination medicament for tropically treating candida mycoses, said medicament being selected from vulvovaginal candidiasis, oropharyngeal candidiasis (oral thrush), napkin dermatitis (napkin thrush) and intertriginous eczema, wherein the epithelial-cell or endothelial-cell adhesion inhibitor is selected from a non-steroidal anti-inflammatory drug (NSAID) having sufficient COX1 inhibition, defined by the exclusion of compounds having a COX1/COX-2 ratio (IC50, microM) of > 20, wherein the combination medicament is an ointment, a cream, a vaginal suppository, a vaginal, buccal or sublingual tablet, a powder, or an aerosol.

2. : Use according to claim 1, **characterised in that** the antifungal active ingredient is an azole or conazole, preferably clotrimazole, bifonazole, miconazole, econazole, isoconazol, itraconazole, fenticonazole, tioconazole, serticonazole, omoconazole, oxiconazole, fluconazole, in particular clotrimazole, a squalene epoxidase inhibitor, preferably naftifine or terbinafine, or a polyene antifungal agent, preferably nystatin, amphotericin B, caspofungin or natamycin, or, ciclopirox, butenafine or flucytosine.

3. : Use according to either claim 1 or claim 2, **characterised in that** the NSAID is selected from indomethacin, mefenamic acid, ketoprofen, acetylsalicylic acid, ibuprofen, lornoxicam, flufenamic acid, diclofenac, piroxicam, bufexamac, etofenamate, felbinac, tenoxicam, pharmaceutically active salts or esters of said substances or mixtures of said substances, in particular diclofenac, ibuprofen or lornoxicam.

4. : Use according to any of claims 1 to 3, **characterised in that** the combination medicament is provided on an inert carrier, in particular on a vaginal ring, a diaphragm or a tampon.

## Revendications

1. Utilisation
- d'un principe actif antimycosique, et
- d'un inhibiteur d'adhésion aux cellules épithéliales ou aux cellules endothéliales pour la fabrication d'un médicament combiné servant au traitement topique de mycoses liées au Candida choisies parmi la candidose vulvovaginale, la candidose oropharyngée (muguet), la dermatite du siège (érythème fessier) et l'eczéma intertrigineux,
dans laquelle l'inhibiteur d'adhésion aux cellules épithéliales ou aux cellules endothéliales est choisi parmi un anti-inflammatoire non stéroïdien (AINS) à inhibition de COX1 suffisante, défini par l'exclusion de composés avec un rapport COX1/COX-2 (IC50, MicroM) > 20, dans laquelle le médicament combiné est un onguent, une crème, un ovule vaginal, un comprimé vaginal, oral ou sublingual, une poudre ou un aérosol.

2. Utilisation selon la revendication 1, **caractérisée en ce que** le principe actif antimycosique est de l'azole ou du conazole, de préférence du clomitrazole, du bifonazole, du miconazole, de l'éconazole, de l'isoconazole, de l'itraconazole, du fenticonazole, du tioconazole, du serticonazole, de l'omoconazole, de l'oxiconazole, de fluconazole, en particulier du clomitrazole, un inhibiteur squalène époxydase,
de préférence de la naftifine ou de la terbinafine, ou
un antimycosique de type polyène, de préférence de la nystatine, de l'amphotéricine B, de la capsofungine ou de la natamycine,
ou du ciclopirox, de la buténafine, de la flucytosine.

3. Utilisation selon la revendication 1 ou 2, **caractérisée en ce que** l'AINS est choisi parmi l'indométhacine, l'acide méfénamique, le kétoprofène, l'acide acétylsalicylique, l'ibuprofène, le lornoxicam, l'acide flufénamique, le diclofénac, le piroxicam, le bufexamac, l'étofénamate, le felbinac, le ténoxicam, des sels ou esters pharmaceutiquement actifs desdites substances ou des mélanges desdites substances, en particulier de diclofénac, d'ibuprofène ou de lornoxicam.

4. Utilisation selon l'une quelconque des revendications 1 à 3, **caractérisée en ce que** le médicament combiné est prévu au niveau d'un support inerte, en particulier au niveau d'un anneau vaginal, d'un diaphragme ou d'un tampon.
